# EUROPEAN PATENT SPECIFICATION

(11) **EP 2 994 093 B1**
(45) Date of publication and mention of the grant of the patent: **12.07.2017**
(21) Application number: 14741529.3
(22) Date of filing: 08.05.2014
(51) Int. Cl.: A61K 8/33, A61Q 13/00, C07C 47/228, C11B 9/00

(54) **3-(4-ISOBUTYL-2-METHYLPHENYL) PROPANAL AS PERFUME INGREDIENT**
3-(4-ISOBUTYL-2-METHYLPHENYL)PROPANAL ALS PARFÜMINHALTSSTOFF
3-(4-ISOBUTYL-2-MÉTHYLPHÉNYL)PROPANAL UTILISÉ COMME INGRÉDIENT DE PARFUM

(30) Priority: 08.05.2013 GB 201308248
(43) Date of publication of application: 16.03.2016
(73) Proprietor: Givaudan SA, 1214 Vernier (CH)
(72) Inventor: GOEKE, Andreas, CH-8400 Winterthur (CH); KRAFT, Philip, CH-8600 Duebendorf (CH); LAUE, Heike, CH-8600 Duebendorf (CH); ZOU, Yue, Shanghai 200433 (CN); VOIROL, Francis, CH-8532 Warth (CH)
(74) Representative: Kraszewska, Agnieszka
(86) International application number: PCT/EP2014/059427
(87) International publication number: WO 2014/180945

(56) References cited:
- GB-A- 988 502
- GB-A- 1 057 360
- US-A- 5 527 769
- SKOUROUMOUNIS GEORGES ET AL: "SYNTHESIS OF 1,3,4,5-TETRAHYDRO-2-BENZOXEPIN DERIVATIVES AS CONFORMATIONALLY RESTRICTED ANALOGUES OF CYCLAMENALDEHYDE-TYPE COMPUNDS AND AS INTERMEDIATES FOR HIGHLY ODOUR-ACTIVE HOMOLOGUES", HELVETICA CHIMICA ACTA, VERLAG HELVETICA CHIMICA ACTA, CH, vol. 79, no. 4, 1 January 1996 (1996-01-01), pages 1095-1109, XP009074278, ISSN: 0018-019X, DOI: 10.1002/HLCA.19960790418

## Description

This invention relates to a perfume ingredient and perfume preparations containing same. In particular, the invention relates to said perfume ingredient or perfume preparations that exhibit muguet (lily of the valley) odour characteristics. Still more particularly, the invention relates to said perfume preparations that contain no, or substantially no, Lilial ^{™}. The invention further relates to methods of making said perfume ingredient and perfume preparations, as well as the use of said perfume ingredient and perfume preparations in fine fragrances and consumer products, such as personal care and household care products. The invention also relates to said fine fragrances and consumer products containing said perfume ingredient and perfume preparations.

Compounds having muguet odour characteristics are very sought after as perfume ingredients. These compounds are important ingredients in floral bases and can act as harmonizers across many types of fragrance creations. Compounds of this type are used widely in personal care and consumer care products, as well as in fine perfumery, to generate pleasant odours or to mask unpleasant odours.

An excellent perfume ingredient widely valued for its muguet odour note is Lilial ^{™} or 3-(4-tert-butylphenyl)-2-methylpropanal (CAS 80-54-6). This compound has found wide use in fine perfumery as well as in personal and household care products. However, its use is controversial in view of recent findings that it exhibits toxic effects on the reproductive organs of male rats and dogs. No effects were found in studies with mice, guinea-pigs and primates, nevertheless, under the Global Harmonized System (GHS) classification system this compound is classified as a CMR2 material. For CMR category 2 materials, it is necessary to establish that quantities proposed for use are harmless to consumers. In view of the regulatory status of Lilial ^{™} it is being replaced with other perfume ingredients.

WO2010105 873 addresses the problem of replacing Lilial ^{™}, the proposed solution residing in the use of mixtures of known ingredients commonly found on the perfumers' palette in order to recreate characteristics substantially similar to those of Lilial ^{™}.

Likewise, WO2009027957 proposes a solution residing in the formulation of combinations of known perfume ingredients from the perfumers' palette.

WO2013045301 also proposes a solution to Lilial ^{™} replacement, which resides in the selection of mixtures of ingredients including the compound Lilyflore ^{™} and a certain indanyl propanal compound, in combination with other secondary perfuming ingredients.

Structure analogues of Lilial ^{™} are disclosed by Winter and Skouroumounis in Helv. Chim. Acta 1996, 79, 1095-1109 and in US 5,527,769, some of them possessing Lilial ^{™} -type odour and also better stability against oxidation to the corresponding propanoic acid.

Furthermore, GB 988502 discloses p-Isobutyldihydrocinnamic aldehyde as substitute for cyclamen aldehyde, also for perfumes of the lily of the valley type, and GB 1057360 is describing several derivatives of dihydrocinnamic aldehyde with alkyl substituents in either *ortho-* or *para*-position useful in perfumery.

The applicant has now found a novel compound that can be employed as a perfume ingredient in perfume compositions and fine fragrances and consumer products. More particularly, the present invention provides said novel compound that possesses desirable muguet odour characteristics. Still more particularly, the applicant has found said novel compound that possesses odour characteristics, which may be perceived and recognised by perfumers as being very reminiscent of the odour of Lilial ^{™} and so can serve as a simple replacement for Lilial ^{™}. Furthermore, the novel compound may have similar or even improved perfume performance compared with Lilial ^{™}. Finally, the applicant has found the novel compound that does not attract the regulatory concerns associated with Lilial ^{™}.

Accordingly, the invention provides in a first aspect a compound according to the formula (I)

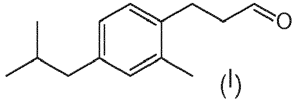

The compound of formula (I) possesses substantially similar odour characteristics and performance characteristics as Lilial ^{™}. As such, and in contradistinction to the prior art proposals related to Lilial ^{™} replacement based on mixtures of known ingredients, the present invention provides for a Lilial ^{™} replacement based on a single compound. This has the obvious advantage of representing a cost-effective solution to the replacement problem, but it is also makes the perfumers' creative process simpler.

The regulatory issues surrounding Lilial ^{™} are born from the fact that it is enzymatically degraded in rats and dogs to tert-butyl benzoic acid (t-BBA), which is known to inhibit glucose synthesis and fatty acid synthesis *in vitro* (McCune et al, Arch Biochem Biophys (1982) 214 (1): 124-133).

tert-butyl benzoic acid is known to cause testicular effects in male rats (Hunter et al. Food Cosmet. Toxicol. 1965, 3: 289-298; Cagen et al. J. Am. Coll. Toxicol. 1989, 8 (5): 1027-1038).

In contrast, the compound of the present invention is not susceptible to enzymatic degradation to its benzoic acid derivative. This was indeed a very surprising result considering the close structural similarity to Lilial ^{™}. The applicant's surprising discovery that an aryl-substituted alkanal containing a methyl substituent on the ring at a position *ortho* to the group bearing the aldehyde functionality is not susceptible to enzymatic degradation to its benzoic acid derivative, provides an insight heretofore not know in the art, and allows perfumers to formulate with a compound that although being structurally similar to Lilial ^{™} (and therefore possessing remarkably similar olfactive properties as these compounds), nevertheless does not raise similar regulatory issues.

In order to study *in vitro* metabolism in rat hepatocytes Lilial ^{™} and the compound of the present invention are incubated in presence of rat hepatocytes in suspension. Decrease of Lilial ^{™} and the compound of the present invention, and formation of the corresponding benzoic acid derivative may be analysed by GC-MS.

Accordingly, in another aspect of the present invention there is provided a compound of formula (I) that forms no, or substantially no, corresponding benzoic acid derivative when incubated with hepatocytes isolated from rats. By "substantially no benzoic acid derivative" is meant that the concentration of said derivative is below the limit of detection, i.e. <1% to 0%. As such, the compound of formula (I) provides perfumers with an eminently suitable surrogate for the valuable yet problematic Lilial ^{™}.

Accordingly, the invention provides in another of its aspects the use of a compound of formula (I) as a perfume ingredient.

The invention provides in another of its aspects the use of a compound of formula (I) in a perfume composition as a replacement for aryl-substituted alkanals, more particularly aryl-substituted propanal odourants that are unsubstituted on the aryl ring at a position *ortho* to the substituent bearing the aldehyde functionality, in particular Lilial ^{™}.

In another aspect of the invention there is provide a method of imparting a muguet odour characteristic to a perfume composition, said method comprising the step of incorporating a compound of formula (I) into said perfume composition.

In yet another aspect of the invention there is provided a perfume composition comprising a compound according to formula (I).

In yet another aspect of the invention there is provided a perfume composition possessing muguet odour characteristics comprising a compound according to the formula (I).

In yet another aspect of the present invention there is provided a perfume composition comprising a compound according to the formula (I) that is free of aryl-substituted alkanal odourants, more particularly aryl-substituted propanal odourants that are unsubstituted on the aryl ring at a position *ortho* to the substituent bearing the aldehyde functionality, in particular Lilial TM.

A perfume composition according to the present invention can be made up entirely by the compound of formula (I). However, a perfume composition may also contain, in addition the compound of formula (I), one or more additional perfume ingredients.

The compound of formula (I) may be present in a perfume composition in any amount depending on the particular olfactive effect that a perfumer wishes to achieve. In a particular embodiment of the present invention, a perfume composition of the present invention may contain a compound of formula (I) in an amount of 0.1 to 100 % by weight of said composition.

If one or more additional perfume ingredients are employed, they may be selected from any known perfume ingredients.

In particular, said perfume ingredients that may be employed in a perfume composition according to the invention include 6-methoxy-2,6-dimethylheptan-1-al (methoxymelonal), 5,9-dimethyl-4,8-decadienal (geraldehyde), beta-methyl-3-(1-methylethyl)benzenepropanal (florhydral), octahydro-8,8-dimethylnaphthalene-2-carbaldehyde (cyclomyral), alpha-methyl-1,3-benzodioxole-5-propionaldehyde (helional), 5-methyl-2-(1-methylbutyl)-5-propyl-1,3-dioxan (Troenan), 3-(o-ethylphenyl)-2,2-dimethylpropionaldehyde (floralozone), farnesol, 3,7,11-trimethyldodeca-1,6,10-trien-3-ol, optionally as an isomeric mixture (nerolidol), 2-methyl-4-phenylbutan-2-ol (dimethylphenylethylcarbinol), cis-4-(isopropyl)cyclohexanemethanol (mayol), 1-(1-hydroxyethyl)-4-(1-methylethyl)cyclohexane (optionally as a mixture of the diastereoisomers) (mugetanol), (4-methyl-3-pentenyl)cyclohexenecarbaldehyde (citrusal), cyclohexyl salicylate, hexyl salicylate, benzyl salicylate, amyl salicylate, 3-(p-(2-methylpropyl)phenyl)-2-methylpropionaldehyde (silvial), 3-p-cumenyl-2-methylpropionaldehyde (cyclamenaldehyde), mixtures of: cis-tetrahydro-2-isobutyl-4-methylpyran-4-ol; trans-tetrahydro-2-isobutyl-4-methylpyran-4-ol; (florol), triethyl citrate and dipropylene glycol.

Said perfume ingredients may additionally include Amyl Salicylate (2050-08-0 ); Aurantiol ® (89-43-0); Benzyl Salicylate (118-58-1); Cis-3-hexenyl Salicylate (65405-77-8); Citronellyl Oxyacetaldehyde (7492-67-3); Cyclemax (7775-00-0); Cyclohexyl Salicylate (25485-88-5); Cyclomyral ® (68738-94-3); Citronellol (106-22-9); Geraniol (106-24-1); Cyclopentol Hc 937165 (84560-00-9); Cymal (103-95-7); Dupical (30168-23-1); Ethyl Linalool (10339-55-6); Floral Super (71077-31-1); Florhydral ® (125109-85-5); Florol ® (63500-71-0); Gyrane (24237-00-1); Hexyl Salicylate (6259-76-3); Helional (TM) (1205-17-0); Hydroxycitronellal (107-75-5); Linalool (78-70-6); Lyral ® (31906-04-4); Majantol ® (103694-68-4); Mayol ® (13828-37-0); Melafleur (68991-97-9); Melonal (106-72-9); Mugetanol (63767-86-2); Muguesia (56836-93-2); Muguet alcohol (13351-61-6); Verdantiol (91-51-0); Peonile ® (10461-98-0); Phenoxanol ® (55066-48-3); Rossitol ® (215231-33-7); Silvial ® (6658-48-6); Suzural (6658-48-6); Muguol ® (18479-57-7); Tetrahydro Linalol (78-69-3); Acalea (84697-09-6); Dihydro Iso Jasmonate (37172-53-5); Hexyl Cinnamic Aldehyde (101-86-0); Hedione ® (24851-98-7); Acetoin (513-86-0); Adoxal (141-13-9); Aldolone ® (207228-93-1); AMBROCENIDE ® (211299-54-6); Ambroxan (3738-00-9); Azurone ® (362467-67-2); Bacdanol ® (28219-61-6); Calone 1951 ® (28940-11-6); Cetalox ® (3738-00-9); Cinnamic alcohol (104-54-1); Citral (5392-40-5); Cyclabute (67634-20-2); Cyclacet (TM) (5413-60-5); Cyclaprop ^{™} (17511-60-3); Cyclohexadecanolide (109-29-5); Cyclohexadecenone (3100-36-5); Cyclopentadecanone (507-72-7); Delta Damascone (57378-68-4); Ebanol ® (67801-20-1); Elintaal Forte (40910-49-4); Ethyl Vanillin (121-32-4); Ethylene Brassylate (105-95-3); Exaltenone 942008 (14595-54-1); Exaltolide Total 935985 (106-02-5); Floralozone (67634-14-4); Fructalate (72903-27-6); Gamma Decalactone (706-14-9); Habanolide (111879-80-2); Helvetolide ® (141773-73-1); Hexamethylindanopyran (1222-05-5); Hydroxyambran ® (118562-73-5); Iso E Super ® (54464-57-2); Iso Hexenyl Cyclohexenyl Carboxaldehyde (37677-14-8); Jasmal (18871-14-2); Javanol ® (198404-98-7); Lauric Aldehyde (112-54-9); Mefranal (55066-49-4); Muscenone (63314-79-4); Tonalid ® (1506-02-1); Nectaryl ® (95962-14-4); Norlim Banol (70788-30-6); Para Hydroxy Phenyl Butanone (5471-51-2); Pino Acetaldehyde (33885-51-7); Romandolide ® (236391-76-7); Sanjinol (28219-61-6); Silvanone ® Supra (109-29- 5/507-72-7); Terpineol (8000-41-7); Vanillin (121-33-5); and Velvione ® (37609-25-9), wherein, the figures in parentheses are CAS numbers.

A perfume composition need not be limited to the perfume ingredients listed above. Other perfume ingredients commonly used in perfumery may be employed, for example any of those ingredients described in "Perfume and Flavour Chemicals". S. Arctander, Allured Publishing Corporation, 1994, IL, USA, which is incorporated herein by reference, including essential oils, plant extracts, absolutes, resinoids, odourants obtained from natural products and the like.

The perfume ingredients contained in said perfume compositions are described above, but of course, the perfume mixture may not be limited to the stated ingredients. In particular, perfume compositions may comprise adjuvants that are commonly employed in perfume compositions. The term "adjuvants" refers to ingredients that might be employed in a perfume composition for reasons not specifically related to the olfactive performance of said composition. For example, an adjuvant may be an ingredient that acts as an aid to processing a perfume ingredient or ingredients, or a composition containing said ingredient(s), or it may improve handling or storage of a perfume ingredient or composition containing same. It might also be an ingredient that provides additional benefits such as imparting colour or texture. It might also be an ingredient that imparts light resistance or chemical stability to one or more ingredients contained in a perfume ingredient or composition containing same. A detailed description of the nature and type of adjuvants commonly used in perfume compositions containing same cannot be exhaustive, but it has to be mentioned that said ingredients are well known to a person skilled in the art. Examples of adjuvants include solvents and co-solvents; surfactants and emulsifiers; viscosity and rheology modifiers; thickening and gelling agents; preservative materials; pigments, dyestuffs and colouring matters; extenders, fillers and reinforcing agents; stabilisers against the detrimental effects of heat and light, bulking agents, acidulants, buffering agents and antioxidants.

Furthermore, any one or more of the perfume ingredients or adjuvants employed in the present invention might be formulated in a delivery vehicle if desired to provide a desired effect. Delivery vehicles may include encapsulates. Alternatively, a delivery vehicle may be in the form of a solid support, e.g. a polymeric support material onto which one or more perfume ingredients or adjuvants may be chemically or physically bound. Still further, one or more perfume ingredients or adjuvants may be dissolved or dispersed in a matrix material, which serves to control the rate at which said ingredient or ingredients emanates therefrom. In yet an alternative embodiment, one or more ingredients or adjuvants may be supported on a porous substrate, such as a cyclodextrin or a zeolite or other inorganic material. In a still further embodiment, one or more perfume ingredients may be provided in the form of a pro-perfume, which will react in a suitable environment to release the perfume ingredient in a controlled manner.

Having regard to the foregoing, it will be appreciated that a perfume composition may be at least partly in solid form, in gel form, in foam form and/or liquid form. If it is present in solid form, it then it may take the form of granules, powders or tablets.

The compound of formula (I), or perfume compositions described herein, may be employed to add a characteristic odour, in particular a muguet odour, to all manner of personal care and household care compositions.

According to another aspect of the present invention there is provided a method of imparting muguet odour characteristics to a composition comprising the step of adding to said composition a compound according to formula (I) or a perfume composition containing said compound.

A compound of formula (I) as a perfume ingredient, or when used in perfume compositions can generate particularly substantive and long-lasting muguet odour characteristics.

The compound of formula (I) is a particularly impactful perfume ingredient. The impact that a perfume ingredient exerts is related to its Odour Value. Odour Value is the ratio of vapour pressure to detection threshold concentration.

The compound of formula (I) has an extremely high Odour Value, specifically 559'071. Related perfume ingredients are not impactful by comparison. For example Lilial ^{™} has an odour value of only 32'978 and Cyclamen aldehyde has an Odour Value of only 21'986.

The remarkably high Odour Value of the compound of formula (I) is significant in that there is a need for sustainability and the provision of impactful perfume ingredients enables perfumers to create desirable fragrance accords with lower concentrations of perfume ingredients.

Consumer products such as personal and household care compositions include, but are not limited to a textile treatment product, an ironing aid, a cleaning cloth, a laundry detergent, a cleaning product, in particular, for hard and/or soft surfaces, a household cleaner, a care product, a wash care product, a laundry care product, a room fragrancer, and air freshener, a conditioner, a colorant, a fabric conditioner, a conditioning substrate, a pharmaceutical, a crop protection product, a polish, a food, a cosmetic product, a fertilizer, a building material, an adhesive, a bleach, a decalcifier, an autocare product, floorcare product, cookercare product, leathercare product or furniture care product, a scourer, a disinfectant, a fragrancer, a mold remover and/or a precursor of the aforementioned products.

The skilled person is fully aware of the applicability of perfume ingredients, and compositions to personal and house hold care compositions and a very detailed description of such compositions is not warranted here. However, specific compositions that can be mentioned include cleaning compositions; autocare compositions; Cosmetic compositions; textile treatment compositions; and air freshener and air care compositions.

Cleaning products include:-
Toilet cleaners or lavatory cleaners, in other words, products for cleaning lavatory bowls and urinals, these products being supplied preferably in the form of powders, blocks, tablets or liquids, preferably gels. Besides other typical ingredients such as surfactants, they generally include organic acids e.g., citric acid and/or lactic acid) or sodium hydrogen sulfate, amidosulfuric acid or phosphoric acid for removing limescale or urine scale;
Pipe-cleaning products or drain cleaners. These are typically strongly alkaline products which serve in general to remove pipe blockages comprising organic materials-such as hair, fat, food residues, soap deposits, and the like. Additions of Al powder or Zn powder may serve for the formation of H2 gas with an effervescence effect. Possible ingredients are commonly alkalis, alkaline salts, oxidizing agents, and neutral salts. Supply forms in powder form preferably also include sodium nitrate and sodium chloride. Pipe-cleaning products in liquid form may preferably also include hypochlorite. There are also enzyme-based drain cleaners as well. Acidic products are likewise possible;
Universal or all-purpose or general-purpose cleaners. These are cleaners which can be used universally for all hard surfaces in the household and in commerce that can be wiped down wet or damp. Generally speaking, they are neutral or slightly alkaline or slightly acidic products, especially liquid products. All-purpose or general-purpose cleaners generally contain surfactants, builders, solvents and hydrotropes, dyes, preservatives, and the like;
All-purpose cleaners with special disinfectant properties. They additionally include active antimicrobial ingredients (e.g., aldehydes, alcohols, quaternary ammonium compounds, amphoteric surfactants, triclosan);
Sanitary cleaners. These are products for cleaning in bath and toilet. The alkaline sanitary cleaners are used preferably for removing fatty soiling, whereas the acidic sanitary cleaners are employed in particular, for removing limescale. Sanitary cleaners advantageously also have a considerable disinfectant action, particularly the strongly alkaline sanitary cleaners that contain chlorine;
Oven cleaners or grill cleaners which may be supplied in the form of gels or foam sprays. They generally serve for removing burnt-on or carbonized food residues. Oven cleaners are preferably given a strongly alkaline formulation using, for example, sodium hydroxide, sodium metasilicate, 2-aminoethanol. In addition they generally contain anionic and/or nonionic surfactants, water-soluble solvents, and, in some cases, thickeners such as polycarboxylates and carboxymethylcellulose;
Metal polishes. These are cleaners for particular types of metal such as stainless steel or silver. Stainless steel cleaners preferably contain, besides acids (preferably up to 3% by weight, e.g., citric acid, lactic acid), surfactants (in particular, up to 5% by weight, preferably nonionic and/or anionic surfactants), and water, solvents as well (preferably up to 15% by weight) to remove fatty soiling, and also further compounds such as thickeners and preservatives. Very fine polishing structures are included, furthermore, in products for preferably bright stainless steel surfaces. Silver polishes, in turn, may be provided in an acidic formulation. In particular, for removing black deposits of silver sulfide they contain, preferably, complexing agents (e.g., thiourea, sodium thiosulfate). Typical supply forms are polishing cloths, dipping baths, pastes, and liquids. Dark discolorations (oxide layers) are removed using copper cleaners and nonferrous-metal cleaners (e.g., for brass and bronze). They generally have a weakly alkaline formulation (preferably with ammonia) and in general contain polishing agents and also, preferably, ammonium soaps and/or complexing agents;
Glass cleaners and window cleaners. These products serve preferably to remove dirt, especially greasy dirt, from glass surfaces. Preferably they contain compounds such as anionic and/or nonionic surfactants (in particular, up to 5% by weight), ammonia and/or ethanolamine (in particular, up to 1% by weight), ethanol and/or 2-propanol, glycol ethers (in particular, 10-30% by weight), water, preservatives, dyes, anti-misting agents and the like; and
Special-purpose cleaning products, examples being those for glass-ceramic hobs, and also carpet cleaners and stain removers.

Autocare products include:-
Paint preservers, paint polishes, paint cleaners, wash preservers, shampoos for auto washing, auto-wash and wax products, polishes for trim metals, protective films for trim metals, plastics cleaners, tar removers, screen cleaners, engine cleaners, and the like.

Cosmetic products include:-
(a) cosmetic skincare products, especially bath products, skin washing and cleansing products, skincare products, eye makeup, lip care products, nail care products, intimate care products, foot care products;
(b) cosmetic products with specific effects, especially sunscreens, tanning products, de-pigmenting products, deodorants, antiperspirants, hair removers, shaving products, perfumes;
(c) cosmetic dental-care products, especially dental and oral care products, tooth care products, cleaners for dental prostheses, adhesives for dental prostheses; and
(d) cosmetic hair care products, especially hair shampoos, hair care products, hair setting products, hair-shaping products, and hair coloring products.

Textile treatment products include:-
Detergents or fabric conditioners, for example, in either liquid or solid form.

Air fresheners and room fragrancers include:-
Products that contain preferably volatile and usually pleasant-smelling compounds which advantageously can even in very small amounts mask unpleasant odours. Air fresheners for living areas contain, in particular, natural and synthetic essential oils such as pine needle oils, citrus oil, eucalyptus oil, lavender oil, and the like., in amounts for example of up to 50% by weight. As aerosols they tend to contain smaller amounts of such essential oils, by way of example less than 5% or less than 2% by weight, but additionally include compounds such as acetaldehyde (in particular, <0.5% by weight), isopropyl alcohol (in particular, <5% by weight), mineral oil (in particular, <5% by weight), and propellants. Other presentation forms include sticks and blocks. They are produced typically using a gel concentrate comprising essential oils. It is also possible to add formaldehyde (for preservation) and chlorophyll (preferably <5% by weight), and also further ingredients. Air fresheners are not, however, restricted to living spaces, but may also be intended for autos, cupboards, dishwashers, refrigerators or shoes, and even their use in vacuum cleaners is a possibility. In the household (e.g., in cupboards), for example, in addition to the odour improvers, disinfectants as well are employed, containing preferably compounds such as calcium phosphate, talc, stearin, and essential oils, these products taking the form, for example, of sachets.

Consumer product compositions referred to hereinabove, particularly those for use in washing or cleaning applications may contain one or more of the following substances:
Builder substances, surfactants, enzymes, bleaching agents, such as preferably organic and/or inorganic peroxygen compounds, peroxygen activators, water-miscible organic solvents, sequestering agents, electrolytes, pH regulators, thickeners, and further adjuvants such as soil release active substances, optical brighteners, graying inhibitors, color transfer inhibitors, foam regulators, and dyes.

Surfactants include anionic surfactants, nonionic surfactants, and mixtures thereof, but also cationic surfactants, are appropriate. Suitable nonionic surfactants are, in particular, ethoxylation and/or propoxylation products of alkyl glycosides and/or of linear or branched alcohols each having 12 to 18 carbon atoms in the alkyl portion and 3 to 20, by preference 4 to 10, alkyl ether groups. Also usable are corresponding ethoxylation and/or propoxylation products of N-alkylamines, vicinal diols, fatty acid esters and fatty acid amides that correspond, in terms of the alkyl portion, to the aforesaid long-chain alcohol derivatives, and of alkylphenols having 5 to 12 carbon atoms in the alkyl residue.

Suitable anionic surfactants include soaps, and those that contain sulfate or sulfonate groups having preferably alkali ions as cations. Soaps include alkali salts of the saturated or unsaturated fatty acids having 12 to 18 carbon atoms. Such fatty acids can also be used in incompletely neutralized form. Included among the usable surfactants of the sulfate type are the salts of the sulfuric acid semi-esters of fatty alcohols having 12 to 18 carbon atoms, and the sulfated products of the aforesaid nonionic surfactants having a low degree of ethoxylation. Included among the usable surfactants of the sulfonate type are linear alkylbenzenesulfonates having 9 to 14 carbon atoms in the alkyl portion, alkanesulfonates having 12 to 18 carbon atoms, and olefinsulfonates having 12 to 18 carbon atoms that are produced upon reaction of corresponding monoolefins with sulfur trioxide, as well as alpha-sulfofatty acid esters that are produced upon sulfonation of fatty acid methyl or ethyl esters.

Cationic surfactants include esterquats and/or the quaternary ammonium compounds (QACs). QACs may be produced by the reaction of tertiary amines with alkylating agents such as methyl chloride, benzyl chloride, dimethyl sulfate, dodecyl bromide, but also ethylene oxide. The alkylation of tertiary amines having a long alkyl residue and two methyl groups occurs particularly easily, and the quaternization of tertiary amines having two long residues and one methyl group can also be carried out using methyl chloride under mild conditions. Amines that possess three long alkyl residues or hydroxy-substituted alkyl residues have low reactivity, and are quaternized, for example, using dimethyl sulfate. Suitable QACs are, for example, benzalkonium chloride (N-alkyl-N,N-dimethylbenzylammonium chloride), benzalkon B (m,p-dichlorobenzyldimethyl-C12-alkylammonium chloride), benzoxonium chloride (benzyldodecyl-bis(2-hydroxyethyl)ammonium chloride), cetrimonium bromide (N-hexadecyl-N,N-trimethylammonium bromide), benzetonium chloride (N,N-dimethyl-N-[2-[2-[p-(1,1,3,3-tetramethylbutyl)phenoxy]ethoxy]ethyl]benzylammonium chloride), dialkyldimethylammonium chlorides such as di-n-decyldimethylammonium chloride, didecyldimethylammonium bromide, dioctyldimethylammonium chloride, 1-cetylpyridinium chloride, and thiazoline iodide, as well as mixtures thereof. Preferred QACs are the benzalkonium chlorides having C8 to C22 alkyl residues, in particular C12 to C14 alkylbenzyldimethylammonium chloride.

Esterquats include the commercially available methylhydroxyalkyldialkoyloxyalkylammonium methosulfates marketed by the Stepan company under the trademark Stepantex^{™}, or the products of Cognis Deutschland GmbH known under the trade name Dehyquat^{™}, or the Rewoquat^{™} products of Goldschmidt-Witco.

Surfactants may be employed in amounts of 5 wt % to 50 wt % in a consumer product of the present invention.

Builders include the water-soluble and/or water-insoluble, organic and/or inorganic builders. In particular, they include the water-soluble organic builder substances are polycarboxylic acids, more particularly citric acid and sugar acids, monomeric and polymeric aminopolycarboxylic acids, in particular methylglycinediacetic acid, nitrilotriacetic acid, and ethylenediaminetetraacetic acid, as well as polyaspartic acid, polyphosphonic acids, in particular aminotris(methylenephosphonic acid), ethylenediaminetetrakis(methylenephosphonic acid), and 1-hydroxyethane-1,1-diphosphonic acid, polymeric hydroxy compounds such as dextrin, as well as polymeric (poly)carboxylic acids, polymeric acrylic acids, methacrylic acids, maleic acids, and mixed polymers thereof, which can also contain small proportions of polymerizable substances having no carboxylic-acid functionality. The relative molecular weight of homopolymers of unsaturated carboxylic acids is generally between 5000 and 200,000, that of the copolymers between 2000 and 200,000, based in each case on free acid. Suitable compounds of this class are copolymers of acrylic acid or methacrylic acid with vinyl ethers, such as vinylmethyl ethers, vinyl esters, ethylene, propylene, and styrene, in which the proportion of acid is equal to at least 50 wt %. It is also possible to use, as water-soluble organic builder substances, terpolymers that contain two unsaturated acids and/or salts thereof as monomers and, as a third monomer, vinyl alcohol and/or a vinyl alcohol derivative or a carbohydrate. The first acid monomer or salt thereof may be derived from an ethylenically mono-unsaturated C3 to C8 carboxylic acid. The second acid monomer or salt thereof can be a derivative of a C4 to C8 dicarboxylic acid, for example maleic acid. The third monomeric unit is constituted by vinyl alcohol and/or an esterified vinyl alcohol. Polymers may contain 60 wt % to 95 wt %, in particular 70 wt % to 90 wt %, (meth)acrylic acid or (meth)acrylate, as well as 5 wt % to 40 wt % vinyl alcohol and/or vinyl acetate. Particular polymers are those in which the weight ratio of (meth)acrylic acid respectively (meth)acrylate to maleic acid or maleate is between 1:1 and 4:1. Both the quantities and the weight ratios are based on the acids. The second acid monomer or salt thereof can also be a derivative of an allylsulfonic acid that is substituted in the 2-position with an alkyl radical, e.g. a C1 to C4 alkyl radical, or with an aromatic radical that may be derived from benzene or benzene derivatives. Terpolymers may contain 40 wt % to 60 wt %, in particular 45 to 55 wt %, (meth)acrylic acid or (meth)acrylate, particularly preferably acrylic acid or acrylate, 10 wt % to 30 wt %, by preference 15 wt % to 25 wt % methallylsulfonic acid or methallylsulfonate, and as a third monomer 15 wt % to 40 wt %, by preference 20 wt % to 40 wt % of a carbohydrate. This carbohydrate can be, for example, a mono-, di-, oligo-, or polysaccharide, e.g. sucrose. The terpolymers generally have a relative molecular weight between 1000 and 200,000. Further copolymers include those that comprise, as monomers, acrolein and acrylic acid/acrylic acid salts, or vinyl acetate. Especially for the manufacture of liquid detergents, the organic builder substances can be used in the form of aqueous solutions, for example a 30- to 50-weight-percent aqueous solutions. All the aforesaid acids may be used in the form of their water-soluble salts, in particular their alkali salts.

Organic builder substances can be employed in quantities of up to 40 wt %.

Water-soluble inorganic builder materials include alkali silicates and polyphosphates, e.g. sodium triphosphate. Crystalline or amorphous alkali aluminosilicates, e.g. crystalline sodium aluminosilicates, may also be employed as water-insoluble, water-dispersible inorganic builder materials, in quantities of up to 50 wt %, for example. Aluminosilicates typically comprise particles having a particle size less than 30 [mu]m.

Crystalline alkali silicates may also be employed, either alone or used with amorphous silicates. The alkali silicates usable in consumer products of the present invention as detergency builders may have a molar ratio of alkali oxide to SiO₂ below 0.95, in particular from 1:1.1 to 1:12, and can be present in amorphous or crystalline fashion. The alkali silicates may be sodium silicates, in particular the amorphous sodium silicates, having a Na₂O:SiO₂ molar ratio from 1:2 to 1:2.8.

Builder substances may be contained in consumer product compositions according to the present invention at levels up to 60 wt %.

Peroxygen compounds include organic peracids or peracid salts of organic acids such as phthalimidopercapronic acid, perbenzoic acid, or salts of diperdodecanedioic acid, hydrogen peroxide, and inorganic salts that release hydrogen peroxide under application conditions, such as perborate, percarbonate, and/or persilicate. If solid peroxygen compounds are to be used, they can be utilized in the form of powders or granulates, which in principle can also be encased in known fashion.

Peroxygen compounds may be employed in amounts up to 50 wt %. The addition of small quantities of known bleaching-agent stabilizers, for example phosphonates, borates respectively metaborates, and metasilicates, as well as magnesium salts such as magnesium sulfate, may be useful.

Compounds that, under perhydrolysis conditions, yield aliphatic peroxocarboxylic acids having preferably 1 to 10 carbon atoms, in particular 2 to 4 carbon atoms, and/or (optionally substituted) perbenzoic acid, can be used as bleach activators. Substances that carry O- and/or N-acyl groups having the aforesaid number of carbon atoms, and/or optionally substituted benzoyl groups, are suitable. Multiple acylated alkylenediamines, in particular tetraacetylethylendiamine (TAED), acylated triazine derivatives, in particular 1,5-diacetyl-2,4-dioxohexahydro-1,3,5-triazine (DADHT), acylated glycolurils, in particular tetraacetyl glycoluril (TAGU), N-acylimides, in particular N-nonanoyl succinimide (NOSI), acylated phenolsulfonates, in particular n-nonanoyl or isononanoyl oxybenzenesulfonate (n- or iso-NOBS), carboxylic acid anhydrides, in particular phthalic acid anhydride, acylated polyvalent alcohols, in particular triacetin, ethylene glycol diacetate, 2,5-diacetoxy-2,5-dihydrofuran, and enol esters, as well as acetylated sorbitol and mannitol respectively mixtures thereof (SORMAN), acylated sugar derivatives, in particular pentaacetylglucose (PAG), pentaacetylfructose, tetraacetylxylose and octaacetyllactose, as well as acetylated, optionally N-alkylated glutamine and gluconolactone, and/or N-acylated lactams, for example N-benzoylcaprolactam, may be employed. Hydrophilically substituted acyl acetates and acyl lactams may likewise be employed. Combinations of conventional bleach activators can also be used. Such bleach activators can be contained in the usual quantity range, by preference in quantities from 1 wt % to 10 wt %, in particular 2 wt % to 8 wt %, based on the entire agent.

In addition to or instead of the aforementioned conventional bleach activators, sulfonimines and/or bleach-intensifying transition metal salts or transition metal complexes can also be contained as bleach catalysts. Included among the appropriate transition metal compounds are, in particular, salen complexes of manganese, iron, cobalt, ruthenium, or molybdenum and nitrogen-analog compounds thereof, carbonyl complexes of manganese, iron, cobalt, ruthenium, or molybdenum, complexes of manganese, iron, cobalt, ruthenium, molybdenum, titanium, vanadium, and copper having nitrogen-containing tripod ligands, amine complexes of cobalt, iron, copper, and ruthenium. Combinations of bleach activators and transition metal bleach catalysts can likewise be used. Bleach-intensifying transition metal complexes, in particular having the central atoms Mn, Fe, Co, Cu, Mo, V, Ti, and/or Ru, can be used in conventional quantities, such as up to 1 wt % based on the weight of the consumer product composition.

Suitable enzymes that may be employed in consumer product compositions are those from the class of the proteases, cutinases, amylases, pullulanases, hemicellulases, cellulases, lipases, oxidases, and peroxidases, as well as mixtures thereof. Enzymatically active substances recovered from fungi or bacteria, such as Bacillus subtilis, Bacillus licheniformis, Streptomyces griseus, Humicola lanuginosa, Humicola insolens, Pseudomonas pseudoalcaligenes, or Pseudomonas cepacia, are also suitable. The enzymes that are used as applicable can be adsorbed onto carrier substances and/or embedded into encasing substances in order to protect them from premature inactivation. They may be contained in washing products according to the present invention in amounts typically below 5 wt %.

Optical brighteners include derivatives of diaminostilbenedisulfonic acid or alkali metal salts thereof. Suitable, for example, are salts of 4,4'-bis(2-anilino-4-morpholino-1,3,5-triazinyl-6-amino)stilbene-2,2'-disulfonic acid, or compounds of similar structure that carry, instead of the morpholino group, a diethanolamino group, a methylamino group, an anilino group, or a 2-methoxyethylamino group. Brighteners of the substituted diphenylstyryl type can also be present, e.g. the alkali salts of 4,4'-bis(2-sulfostyryl)diphenyl, of 4,4'-bis(4-chloro-3-sulfostyryl)diphenyl, or of 4-(4-chlorostyryl)-4'-(2-sulfostyryl)diphenyl. Mixtures of the aforesaid optical brighteners can also be used.

Foam inhibitors include organopolysiloxanes and mixtures thereof with microfine, optionally silanated silicic acid, as well as paraffin waxes and mixtures thereof with silanated silicic acid or bis-fatty acid alkylenediamides. Mixtures of different foam inhibitors, for example those made of silicones, paraffins, or waxes, can also be employed. The foam inhibitors, in particular silicone- and/or paraffin-containing foam inhibitors are by preference bound to a granular carrier substance that is soluble or dispersible in water. Mixtures of paraffins and bistearylethylenediamide, in particular may be employed.

Soil release active substances are those compounds that positively influence the ability of oils and fats to be washed out of textiles. This effect becomes particularly apparent when the soiled textile is one that has already been previously washed several times with a washing agent according to the present invention that contains this oil- and fat-releasing component. The preferred oil- and fat-releasing components include, for example, nonionic cellulose ethers such as methyl cellulose and methylhydroxypropyl cellulose having a 15 to 30 wt % proportion of methoxy groups and a 1 to 15 wt % proportion of hydroxypropoxyl groups, based in each case on the nonionic cellulose ethers, as well as polymers, known from the existing art, of phthalic acid and/or terephthalic acid resp. of their derivatives with monomeric and/or polymeric diols, in particular polymers of ethylene terephthalates and/or polyethylene glycol terephthalates or anionically and/or nonionically modified derivatives thereof.

Colour transfer inhibitors include polymers of vinylpyrrolidone, vinylimidazole, vinylpyridine-N-oxide, or copolymers thereof. Also usable are both polyvinylpyrrolidones having molecular weights from 15,000 to 50,000 and polyvinylpyrrolidones having molecular weights above 1,000,000, in particular from 1,500,000 to 4,000,000, N-vinylimidazole/N-vinylpyrrolidone copolymers, polyvinyloxazolidones, copolymers based on vinyl monomers and carboxylic acid amides, pyrrolidone-group-containing polyesters and polyamides, grafted polyamidoamines and polyethylenimines, polymers having amide groups made up of secondary amines, polyamine-N-oxide polymers, polyvinyl alcohols, and copolymers based on acrylamidoalkenyl sulfonic acids. It is also possible, however, to use enzymatic systems encompassing a peroxidase and hydrogen peroxide or a substance that yields hydrogen peroxide in water.

Graying inhibitors are those materials that keep dirt that has been detached from the textile fibers suspended in a washing medium. Water-soluble colloids, usually organic in nature, are suitable for this, for example starch, size, gelatin, salts of ethercarboxylic or ethersulfonic acids of starch or of cellulose, or salts of acid sulfuric acid esters of cellulose or of starch. Water-soluble polyamides containing acid groups are also suitable for this purpose. Starch derivatives other than those recited above can also be used, for example aldehyde starches. Cellulose ethers such as carboxymethyl cellulose (sodium salt), methyl cellulose, hydroxyalkyl cellulose, and mixed ethers such as methylhydroxyethyl cellulose, methylhydroxypropyl cellulose, methylcarboxymethyl cellulose, and mixtures thereof may be used, for example in quantities from 0.1 to 5 wt % based on the weight of the consumer product.

Organic solvents include alcohols having 1 to 4 carbon atoms, in particular methanol, ethanol, isopropanol, and tert-butanol, diols having 2 to 4 carbon atoms, in particular ethylene glycol and propylene glycol, as well as mixtures thereof, and the ethers derivable from the aforesaid compound classes. Water-miscible solvents of this kind are present in washing products according to the present invention in amounts typically not exceeding 30 wt %.

pH regulators include citric acid, acetic acid, tartaric acid, malic acid, lactic acid, glycolic acid, succinic acid, glutaric acid, and/or adipic acid, but also mineral acids, in particular sulfuric acid, or bases, in particular ammonium hydroxides or alkali hydroxides. pH regulators of this kind are contained in the agents according to the present invention in quantities preferably not above 20 wt %, in particular from 1.2 wt% to 17 wt %.

The compound of the present invention may be particularly used to perfume household products containing enzymes, such as those defined above, and in particular textile treatment products, such as detergents, containing enzymes. There now follows a series of examples that serve to further illustrate the invention.

### Example 1

### Synthesis of 3-(4-isobutyl-2-methylphenyl) propanal

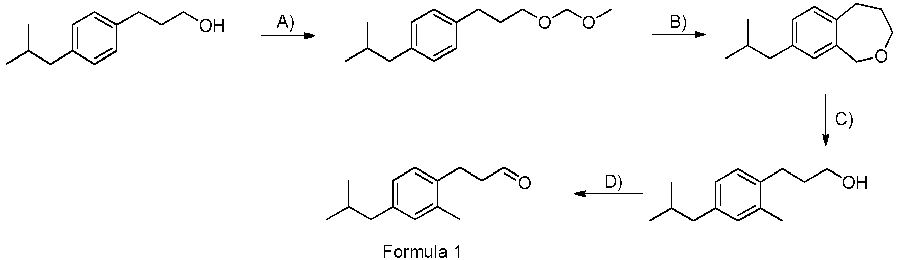

### A) 1-Isobutyl-4-(3-(methoxymethoxy)propyl)benzene:

To a solution 3-(4-isobutylphenyl)propan-1-ol (641.0 g, 3.33 mol) of in 1.5 L of dimethoxymethane was added lithium bromide (14.5 g) and p-toluene sulfonic acid (10.0 g). The mixture was stirred for 20 h at room temperature and then poured with stirring into aqueous sodium hydroxide (200 ml, 2M). The organic phase was washed neural with diluted solution of NaCl, dried (MgSO₄) and evaporated in vacuo to yield 1-isobutyl-4-(3-(methoxymethoxy)propyl)benzene (721.0 g, 91.5%) as a colourless oil. ¹H-NMR (400 MHz, CDCl3): δ = 7.17-7.08 (m, 4H), 4.68 (s, 2H), 3.59 (t, *J* = 6.4 Hz, 2H), 3.42 (s, 3H), 2.74 (t, *J* = 7.8 Hz, 2H), 2.49 (d, *J* = 7.1 Hz, 2H), 2.0-1.84 (m, 3H), 0.95 (d, J = 6.6 Hz, 6H) ppm. ¹³C-NMR (400 MHz, CDCl3): δ = 139.5 (s), 139.4 (s), 129.5 (d, 2C), 128.5 (d, 2C), 96.9 (t), 67.6 (t), 55.6 (q), 45.5 (t), 32.5 (t), 31.9 (t), 30.7 (d), 22.8 (q, 2C) ppm. GC/MS (EI): 236 (M⁺), 204 (29), 161 (65), 147 (59), 131 (100), 117 (49), 105 (37), 104 (26), 91 (46), 57 (29), 45 (64).

### B) 8-Isobutyl-1,3,4,5-tetrahydrobenzo[c]oxepine

To a cooled solution (0 °C) of AlCl₃ (10.33 g) in dichlormethane (80 ml) was added dropwise a solution of 1-isobutyl-4-(3-(methoxymethoxy)propyl)benzene (13.9 g, 55.36 mmol) in dichloromethane (40 ml). After the addition was completed, the mixture was stirred for 1.5 h at 5 °C and poured into a aqueous sodium hydroxide solution (80 ml, 2M). The mixture was extracted with MTBE and the organic layers were washed with ater and brine, dried (MgSO₄) and evaporated in vacuo to afford a clear yellowish oil (11.91 g) which was first distilled in Kugelrohr (125 °C, 0.12 mbar) and then purified by chromatography to yield 8-isobutyl-1,3,4,5-tetrahydrobenzo[c]oxepine (6.95, 61%) as a colourless oil. Odor: ¹H NMR (400 MHz, CDCl₃): δ = 7.11 (d, *J* = 7.33 Hz, 1H), 7.01-6.97 (m, 2H), 4.66 (s, 2H), 4.09-4.06 (m, 2H), 3.02-2.98 (m, 2H), 2.46 (d, *J* = 7.33 Hz, 2H), 1.93-1.82 (m, 1H), 0.94 (d, *J* = 6.8 Hz, 6H) ppm. ¹³C-NMR (400 MHz, CDCl₃): δ = 140.3 (s), 140.05 (s), 139.9 (s), 129.8 (d), 129.3 (d), 128.8 (d), 76.1 (t), 75.6 (t), 45.3 (t), 35.5 (t), 30.9 (t), 30.6 (d), 22.8 (q, 2C) ppm. GC/MS (EI): 204 (M⁺, 33), 161 (100), 147 (44), 143 (58), 131 (31), 129 (28), 119 (61), 115 (34), 105 (43), 91 (34).

### C) 3-(4-Isobutyl-2-methylphenyl)propan-1-ol

A catalytic amount of borontriflouride ether complex (0.3 g) was added to a suspension of Pd/C (10%, 0.5 g) and 8-isobutyl-1,3,4,5-tetrahydrobenzo[c]oxepine (50.0 g, 244.5 mmol). The mixture was hydrogenated in an autoclave for 2h at 9 bar and 50 °C for 2h. The suspension was filtered and thin film distilled (160 °C, 0.11 mbar) to yield 3-(4-isobutyl-2-methylphenyl)propan-1-ol (45.39 g, 89.9%) as a viscous colourless oil. Odour: ¹H-NMR (400 MHz, CDCl3): δ = 7.10 (d, *J* = 7.6 Hz, 1H), 6.99-6.94 (m, 2H), 3.75 (t, *J* = 6.6 Hz, 2H), 2.74-2.68 (m, 2H), 2.47 (d, *J* = 7.3 Hz, 2H), 2.34 (s, 3H), 2.19 (s, 1H, -OH), 1.95-1.84 (m, 1H), 0.96 (d, *J* = 6.6 Hz, 6H) ppm. ¹³C-NMR (400 MHz, CDCl3): δ = 139.7 (s), 137.6 (s), 135.9 (s), 131.5 (d), 128.9 (d), 127.1 (d), 63.0 (t), 45.4 (t), 33.5 (t), 30.6 (d), 29.5 (t), 22.9 (q), 19.7 (q) ppm. GC/MS (EI): 206 (M⁺, 25), 163 (100), 161 (27), 145 (84), 119 (53), 117 (33), 115 (32), 105 (41), 91 (40), 41 (23).

### D) 3-(4-Isobutyl-2-methylphenyl)propanal

Pyridinium chlorochromate (PCC, 37.6g, 174.49 mmol) was added portionwise to a solution of 3-(4-isobutyl-2-methylphenyl)propan-1-ol (30.0 g, 145.41 mmol) in dichloro methane (300 ml); the temperature rose to 35 °C. The mixture was stirred for 1h and another amount of PCC (10 g, 46.4 mmol) was added and stirring was continued for another 15 min. The reaction mixture was filtered over Florisil and silica gel. The filtrate was evaporated in vacuo (23.2g) and distilled in kugelrohr (143 °C, 0.08 mbar) to yield 3-(4-isobutyl-2-methylphenyl)propanal (19.01 g, 64%) as a colorless oil. Odor: floral, aldehydic, green, rubbery, Lilial, watery. ¹H-NMR (400 MHz, CDCl3): δ = 9.88 (t, *J* = 1.5 Hz, 1H), 7.07 (d, *J* = 7.6 Hz, 1H), 7.0-6.95 (m, 2H), 2.98-2.93 (m, 2H), 2.79-2.74 (m, 2H), 2.46 (d, *J* = 7.1 Hz, 2H), 2.33 (s, 3H), 1.95-1.82 (m, 1H), 0.95 (d, *J* = 6.6 Hz, 6H) ppm. ¹³C-NMR (400 MHz, CDCl3): δ = 202.2 (d), 140.2 (s), 136 (s), 135.9 (s), 131.6 (d), 128.6 (d), 127.3 (d), 45.4 (t), 44.6 (t), 30.6 (d), 25.5 (t), 22.9 (q), 19.7 (q) ppm. GC/MS (EI): 204 (M⁺, 23), 161 (100), 147 (26), 143 (49), 119 (84), 118 (34), 117 (33), 115 (33), 105 (59), 91 (36).

### Example 2

In this floral perfume formulation 3-(4-isobutyl-2-methylphenyl)propanal increases the floral character and makes this chypre composition fresher with a pleasnt floral tonality, emphasizing the softness of the woods whilst maintaining a fruity spicey character.

| | | |
|---|---|---|
| ETHYL PHENYL ALCOHOL | 60-12-8 | 20.00 |
| ALD C 11 UNDECYLENIQUE at 10% in BB | | 9.00 |
| AMBERMAX 10%/TEC | | 3.00 |
| BENZYL BENZOATE | 120-51-4 | 93.00 |
| BERGAMOTE ESS ITALIE ORPUR | 8007-75-8 | 75.00 |
| CASHMERAN | 33704-61-9 | 9.00 |
| CHENE EXTRAIT CO2/ETHANOL | | 8.00 |
| CITRON ESS ITALIE SFUMATRICE ORPUR | 8008-56-8 | 45.00 |
| DIHYDRO ISOJASMONATE METHYLE | 37172-53-5 | 100.00 |
| GALBANUM ESS CONCENTREE 10% in DPG | | 25.00 |
| GERANIOL 980 | 106-24-1 | 15.00 |
| GIVESCONE | 57934-97-1 | 100.00 |
| GRAPEFRUIT ESS COSMOS | 8016-20-4 | 30.00 |
| JASMIN ABS COMMUNELLE | 8022-96-6 | 10.00 |
| JAVANOL | 198404-98-7 | 2.00 |
| KOHINOOL 862107-86-6 | | 20.00 |
| NONADIENOL-2,6 10%/TEC at 1% in BB | | 15.00 |
| OENANTHATE ALLYLE at 10% in BB | | 8.00 |
| PATCHOULI ESS FRACTION INDONESIE ORPUR | 8014-09-3 | 20.00 |
| PECHE PURE | 104-67-6 | 3.00 |
| PEPPERWOOD | 67643-70-3 | 55.00 |
| SYLKOLIDE | 676532-44-8 | 70.00 |
| VERTOFIX COEUR | 32388-55-9 | 120.00 |
| 3-(4-ISOBUTYL-2-METHYLPHENYL)PROPANAL | | 45.00 |
| | | 900.00 |

### Example 3

*In vitro* metabolism study. A comparison of a compound of formula (I) and Lilial ^{™}.

Cryopreserved hepatocytes from male rats (Sprague Dawley; Lifetechnologies) were defrozen, washed in Cyropreserved Hepatocytes Recovery Medium (CHRM; Lifetechnologies) and suspended in Williams E Medium (WEM; Lifetechnologies). Lilial^{™}, or the compound of formula (I) (final concentration: 100 µM) were added to the cells (1 x 10⁶ viable cells/ml) and suspensions were incubated up to 4 hours at 37°C on a shaker in duplicate. Metabolism of testosterone was monitored as positive control. Decrease of the test compounds and formation of the corresponding benzoic acid derivative was determined by GC-MS analysis of methyl-esters formed after derivatisation with trimethylsilyl-diazomethane (Sigma-Aldrich) in methanol. The test compounds react with diazomethane yielding a methyl-ketone which was used for the quantification of Lilial ^{™} and the compound of formula (I). Metabolism was stopped with ice cold 1 M HCl, samples were extracted with tert-butyl methyl ether (MTBE) and analysed by GC-MS. Incubations containing testosterone as control were also stopped with ice cold 1 M HCl, centrifuged to separate the cells, filtrated and the decrease of testosterone analysed by LC-MS. To quantify decrease of the test substances and formation of benzoic acid metabolites, calibration curves of reference materials (Lilial ^{™} and the compound of formula (I), tert-butyl benzoic acid (Fluka) was prepared in hepatocyte incubation medium and analysed like the hepatocyte samples.

A rapid decrease of testosterone as positive control was observed indicating that the hepatocytes were metabolically active. The compound of formula (I) and Lilial ^{™} were metabolised rapidly in rat hepatocytes and no residual compound was measured after 4 h. Whereas tert-butyl benzoic acid was detected as metabolite of Lilial^{™} (3.4-3.9 µM) no benzoic acid derivative was formed from compound of formula I (Table 1). Limit of detection was <1 µM.

**Table 1. Concentrations of Lilial ^{™} and a compound of formula (I) and corresponding benzoic acid metabolites in rat hepatocytes within 4 hours incubation. Initial test concentration at 0 hours incubation was 100 µM.**

| Compound tested | Residual test compound | Benzoic acid derivative |
|---|---|---|
| Compound of formula (I) | 0 µM | not found |
| Lilial^{™} | 0 µM | 3.4-3.9 µM |

Therefore, the methyl substitution at the benzene ring of the compound of formula (I) prevents the formation of the corresponding benzoic acid derivative *in vitro*. Since benzoic acid derivatives such as tert-butyl benzoic acid from Lilial ^{™} cause reproductive toxicity in male rats, these toxic effects in male rats are prevented by the ortho-substituent of the compound of formula (I).

## Claims

1. A compound according to the formula (I)

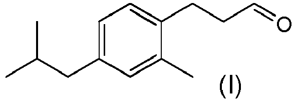

2. The use of a compound according to formula (I) as a perfume ingredient.

3. The use of a compound according to claim 2, wherein the perfume ingredient has muguet odour characteristics.

4. A perfume composition comprising a compound according to the formula (I).

5. A perfume composition according to claim 4 that is free of aryl-substituted alkanal odourants, more particularly aryl-substituted propanals, that are unsubstituted on the aryl ring at a position ortho to the substituent bearing the aldehyde functionality, in particular Lilial ^{™}.

6. A perfume composition according to claim 4 or claim 5 comprising one or more additional fragrance ingredients.

7. A perfume composition according to any of the claims 4 through 6, which exhibits muguet odour characteristics.

8. A personal care or household care composition comprising a compound of formula (I) according to claim 1, or a perfume composition as defined in any of the claims 3 through 7.

9. A method of producing a muguet odour characteristic to a perfume composition comprising the step of adding to said composition a compound defined in claim 1.

## Patentansprüche

1. Verbindung gemäß der Formel (I)

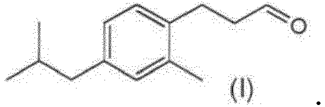

2. Verwendung einer Verbindung gemäß Formel (I) als Parfüm-Inhaltsstoff.

3. Verwendung einer Verbindung nach Anspruch 2, wobei der Parfüm-Inhaltsstoff Muguet-Geruchseigenschaften aufweist.

4. Parfümzusammensetzung, umfassend eine Verbindung gemäß der Formel (I).

5. Parfümzusammensetzung nach Anspruch 4,, die frei ist von arylsubstituierten Alkanal-Geruchsstoffen, insbesondere arylsubstituierten Propanalen, die am Arylring an einer zu dem Substituenten, der die Aldehyd-Funktionalität trägt, ortho-ständigen Position unsubstituiert sind, insbesondere Lilial^{™}.

6. Parfümzusammensetzung nach Anspruch 4 oder 5, umfassend einen oder mehrere andere Duftstoff-Inhaltsstoffe.

7. Parfümzusammensetzung nach einem der Ansprüche 4 bis 6, die Muguet-Geruchseigenschaften zeigt.

8. Körperpflege- oder Haushaltspflegezusammensetzung, umfassend eine Verbindung der Formel (I) nach Anspruch 1 oder eine Parfümzusammensetzung gemäß einem der Ansprüche 3 bis 7.

9. Verfahren zum Versehen einer Parfümzusammensetzung mit einer Muguet-Geruchseigenschaft, bei dem man eine Verbindung gemäß Anspruch 1 zu der Zusammensetzung gibt.

## Revendications

1. Composé répondant à la formule (I)

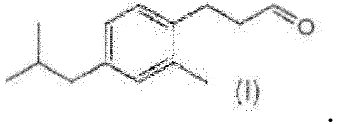

2. Utilisation d'un composé répondant à la formule (I), comme ingrédient de parfum.

3. Utilisation d'un composé selon la revendication 2, dans laquelle l'ingrédient de parfum possède des caractéristiques d'odeur de muguet.

4. Composition de parfum comprenant un composé répondant à la formule (I).

5. Composition de parfum selon la revendication 4, qui est dépourvue d'odorisants de type alcanal substitué par aryle, plus particulièrement de propanals substitués par aryle, qui sont non substitués sur le cycle aryle au niveau d'une position ortho par rapport au substituant portant la fonctionnalité aldéhyde, en particulier Lilial^{™}.

6. Composition de parfum selon la revendication 4 ou la revendication 5, comprenant un ou plusieurs autres ingrédients de fragrance.

7. Composition de parfum selon l'une quelconque des revendications 4 à 6, qui présente des caractéristiques d'odeur de muguet.

8. Composition de soin personnel ou de produit ménager comprenant un composé de formule (I) selon la revendication 1, ou composition de parfum telle que définie selon l'une quelconque des revendications 3 à 7.

9. Méthode de production d'une caractéristique d'odeur de muguet dans une composition de parfum, comprenant l'étape consistant à ajouter un composé défini selon la revendication 1 à ladite composition.
